# EUROPEAN PATENT APPLICATION

(11) **EP 2 777 691 A1**
(43) Date of publication of application: **17.09.2014**
(21) Application number: 13159275.0
(22) Date of filing: 14.03.2013
(51) Int. Cl.: A61K 9/00, A61K 47/10

(54) **Taxoid - Purification of Liquid Excipients**

(71) Applicant: PHARMACHEMIE B.V., 2031 GA Haarlem (NL)
(72) Inventor: Van Lamoen, Richard, 2631 LH Nootdorp (NL); Feunekes, Lendert, 2182 GJ Hillegom (NL); Boing, Jan, 1161 VJ Zwanenburg (NL); Brandsma, Jelte, 2331 CB Leiden (NL); Van Achthoven, Erwin, 2353 EM Leiderdorp (NL); Smit, Ruud, 1985 EL Driehuis (NL)
(74) Representative: Nachshen, Neil Jacob

(57) **Abstract**

The invention relates to taxoid compositions having improved stability.

## Description

The present invention relates to improved stable formulations of taxoid drugs, in particular of docetaxel.

### Background of the Invention

Since their entry into clinical medicine, taxoid drugs (or taxanes) have been formulated in hydrophobic systems. Taxol® (paclitaxel) is sold as a concentrated vial for dilution in a Cremophor/anhydrous ethanol and citric acid whereas Taxotere® (docetaxel) is sold as a "concentrate for solution for infusion" in polysorbate/anhydrous ethanol and citric acid. The stability of the active ingredient in such systems has been a concern and various solutions have been proposed.

EP593656A describes perfusion solutions prepared from paclitaxel or docetaxel containing polysorbate with and without ethanol.

EP645145 examined the effect of processing commercial grade Cremophor by subjecting it to an extra chromatography step (aluminium oxide column). The effect was to reduce the peroxide value, acid value and potassium ion concentration. These factors were then tested for their influence on the stability of paclitaxel compositions. None of peroxide value, acid value or potassium ion concentration were shown to correlate with improved stability rather an effect was observed linking the presence of carboxylic acid anions with decreased stability. The addition of mineral acid such as HCl or use of processed Cremophor (with reduced carboxylate anions) was shown to increase stability of paclitaxel with respect of Baccatin III and the Taxol ethyl ester.

WO9412030 discloses the use of citric acid in taxol compositions (Cremophor and ethanol) to increase stability by adjusting the pH to between 1 and 8.

WO2006133510 discloses docetaxel formulations containing polysorbate 80, anhydrous ethanol, citric acid and a glycol such as polyethylene glycol 300 as having improved stability.

WO2008087076 discloses stabilised taxoid formulations containing polysorbate 80 having reduced levels of water moisture and peroxide, ethanol and anhydrous citric acid. The citric acid is essential for the stability of the composition on storage.

Despite the many suggestions made in the art, the marketed formulations remain substantially unchanged. Thus, Taxotere® is currently presented as an injection concentrate as a sterile, non-pyrogenic, pale yellow to brownish-yellow solution at 20 mg/mL concentration. Each mL of 1-vial Taxotere® contains 20 mg docetaxel (anhydrous) in 0.54 grams polysorbate 80, 0.395 grams dehydrated alcohol and approximately 0.5 mg/mL of Citric Acid. Upon storage, such compositions have been observed to contain degradation products such as e.g. 10-Oxo-docetaxel and 7-Epi-docetaxel.

### Summary of the Invention

The present inventors have surprisingly observed that pretreatment of polysorbate has an unexpected and dramatic effect on the stability of docetaxel and other taxoid drugs such as cabazitaxel. The present invention therefore relates to a pharmaceutical composition of a taxoid drug, polysorbate and optionally, anhydrous ethanol, wherein the content of sodium and potassium ions present in the polysorbate 80 have each been reduced to less than 25ppm. Use of such polysorbate has the surprising effect of not requiring the addition of any pH lowering agent such as citric acid. Preferably, the taxoid drug is docetaxel.

The invention therefore provides improved docetaxel/cabazitaxel compositions that are simpler to manufacture and retain or improve the stability of the marketed product.

The present invention further relates to a method of preparing a docetaxel/cabazitaxel composition containing docetaxellcabazitaxel , polysorbate 80 and optionally anhydrous alcohol wherein the polysorbate 80 has a reduced level of sodium and potassium ions.

### Detailed Description of the Invention

The embodiments of the invention are set out with reference to docetaxel as the taxoid drug but they equally apply to when cabazitaxel is the taxoid drug.

In a first aspect, the present invention relates to a pharmaceutical composition containing docetaxel and polysorbate 80 wherein the level of sodium ions and potassium ions present in the polysorbate 80 is each less than 25ppm.

In a second aspect, the invention relates to a pharmaceutical composition containing docetaxel and polysorbate 80, wherein said polysorbate 80 is substantially free of sodium and potassium metals, preferably less than 10 ppm of each individually.

A third aspect of the invention relates to a stable pharmaceutical composition containing docetaxel that is free of citric acid (or similar pH reducing agents). Preferably, the composition further contains polysorbate 80 and ethanol.

The taxoid composition can be prepared either with anhydrous alcohol or without. Some compositions have no need for the addition of anhydrous alcohol, others can be prepared as a "two-vial" product i.e. one vial containing the taxoid drug and polysorbate 80 and the other, the anhydrous alcohol, preferably ethanol to be added to the first vial prior to use. In each aspect, it is preferred that the composition contains anhydrous alcohol, more preferably, anhydrous ethanol.

The three aspects of the invention may be dependent on one another. Furthermore, with respect to the above first, second and third aspects of the invention, the following preferred embodiments are applicable;

In each aspect, it is preferred that the composition contains anhydrous alcohol, more preferably, anhydrous ethanol.

Preferably, the level of each of the sodium and potassium ions present in the polysorbate 80 is less than 10ppm. In an alternative preference, the pH of the polysorbate 80 is less than 5, more preferably, 3.8 or less, more preferably, 3.8.

In a preferred embodiment of the above, the peroxide level of the polysorbate 80 between 2 and 0.1 or 0.

A particular advantage of the described pharmaceutical compositions is their stability wherein the level of 7-epi-docetaxel is 0.3% or less after storage for 6 months at 25°C and a relative humidity of 60%. More preferably, the level of 7-epi-docetaxel is 0.1 % or less after storage for 6 months at 25°C and a relative humidity of 60%.

In the preferred embodiment wherein the peroxide level of the polysorbate 80 is between 2 and 0.1, the level of 7-epi-docetaxel is 0.3% or less and the level of 10-oxo-docetaxel is 0.4% or less after storage for 6 months at 25°C and a relative humidity of 60%, more preferably, wherein the level of 7-epi-docetaxel is 0.1 % or less and the level of 10-oxo-docetaxel is 0.2% or less after storage for 6 months at 25°C and a relative humidity of 60%.

A further advantage of the composition of the present invention is that, in contrast to stable docetaxel compositions described in the prior art, those described above are stable even in the absence of a pH reducing agent, preferably in the absence of citric acid. The presence of citric acid in a stable docetaxel composition is therefore optional.

In a further preferred embodiment, the pharmaceutical composition has a pH of from 3.0 to 4.5.

A further aspect of the invention relates to a process of preparing a pharmaceutical composition as defined above comprising;
a) exposing polysorbate 80 to cation exchange resin,
b) filtering said mixture to obtain polysorbate 80 with a reduced level of sodium and potassium ions
c) combining said polysorbate 80 with docetaxel which may optionally be dissolved in ethanol.

Preferably, the polysorbate 80 is exposed to resin until the pH of the polysorbate is pH 5.0 or less, more preferably until the pH of the polysorbate is pH 3.8.

Preferably, the polysorbate 80 is of the quality wherein the peroxide level of the polysorbate 80 is between 2 and 0.1.

Preferably, the composition is a stable pharmaceutical composition containing docetaxel that is free of citric acid (or similar pH reducing agents).

In each and every embodiment of the present invention, the preferred anhydrous alcohol is anhydrous ethanol. All references to "alcohol" or "ethanol" mean anhydrous alcohol or ethanol. Preferably, the cation exchange is continued until the resultant polysorbate 80 demonstrates a pH of less than 5.0, more preferably less than 4.0, most preferably a pH of 3.8.

Any polysorbate 80 can be used in the present invention. In certain preferred embodiments, polysorbate 80 with a reduced peroxide level is used. Such reduced peroxide polysorbate 80 is available commercially as peroxide level is indicated on the Certificate of Analysis. The peroxide level can therefore be 0 as indicated by said manufacturers. A suitable peroxide level considered to be "reduced" would be between 2 and 0.1 or between 2 and 0. Examples of suitable low peroxide polysorbate 80 includes; Croda TweenTM 80HP LQ available from Croda (www.croda.com) and Seppic Montanox PPI available from Seppic (www.seppic.com).

Any strong acid cation exchange resin can be used in processing the polysorbate. Suitable cation exchange resins include those sold by Dowex listed at www.dowwaterandprocess.com/products/sac.htm in particular, the Dowex monosphere C range such as Monosphere 650C UPW (polystyrene DVB gel matrix) or the Dowex Marathon C range (styrene DVB gel matrix).

The processing of the polysorbate 80 is preferably carried out after heating the polysorbate 80 to decrease the viscosity thereby enhancing the ion exchange. Typically, the polysorbate 80 is heated to a temperature of between 40-60°C, preferably between 40-50°C.

As used herein all reference to pH measurement refers to the use a pH measurement taken by diluting the substance for testing (polysorbate 80 of composition) with 10 volumes of distilled water and measuring pH in a conventional manner.

The compositions of the present invention can be prepared in any desired concentration of docetaxel. Typically, such compositions contain from 10mg/ml to 160mg/ml of docetaxel and contain docetaxel and polysorbate in a ratio of 1:26 (w/v). For example a 20mg/ml docetaxel solution will preferably contain 529 mg/ml polysorbate 80, a 40mg/ml solution, 723mg/ml polysorbate etc.. The amount of ethanol included in each formulation is typically sufficient to enable the docetaxel to be in solution. For a 20mg/ml composition this may be 15-25 times (w/v) as the amount of docetaxel preferably 20 times. For a composition containing 25-30mg/ml docetaxel, the amount of ethanol may be 5-15 times as much, preferably 9-10 times as much. Preferably, compositions containing 160mg/ml docetaxel may preferably not contain any ethanol.

Compositions wherein cabazitaxel is the active ingredient are typically in the form of a concentrate for injection e.g. containing 60mg cabazitaxel in 1.5 ml polysorbate 80, which may be combined with a diluent e.g. approximately 5.7 mL of 13% (w/w) ethanol in water for injection.

The docetaxel may be in any chemical form e.g. it may be present as docetaxel, anhydrous docetaxel or docetaxel trihydrate.

The cabazitaxel may be in any chemical form e.g. it may be present as Cabazitaxel, anhydrous cabazitaxel or cabazitaxel acetone solvate.

The compositions of the invention therefore represent a significant benefit to the industry. Improved long-term stability of docetaxel compositions has been desired by the industry for a long time. Furthermore, the improved stability has been achieved in a manner that negates the perceived necessity for a pH reducing agent such as citric acid.

The compositions of the present invention have use in the treatment of tumors as set out in the approved indications for Taxotere® such as breast cancer, non-small cell lung cancer, prostate cancer, head and neck cancer and gastric adenocarcinoma as monotherapy or as part of combination therapy as described in the Taxotere® product label for example, (http://www.medicines.org.uk/EMC/medicine/25454/SPC/Taxotere+20mg+ 1ml+concentrate+for+solution+for+infu sion/).

### Examples

### Method and Materials;

Unless specifically stated to the contrary, all docetaxel compositions used in the Examples comprised 20mg/ml docetaxel, 529mg/ml polysorbate 80 and 395mg/ml ethanol.

The levels of alkali metals may be performed using ICP-AES atomic emission spectrometry (inductively coupled plasma atomic emission spectrometry). Such methods are described in Inductively Coupled Plasma/Optical Emission Spectrometry" Xiandeng Hou and Bradley T. Jones in Encyclopedia of Analytical Chemistry R.A. Meyers (Ed.) pp. 9468-9485, John Wiley & Sons Ltd, Chichester, 2000, "Overview Of An Inductively Coupled Plasma (lcp) System" International Journal of Chemical Research Vol. 3, Issue 2, 2011, pp-41-48 http://www.bioinfo.in/contents.php?id=23 or "Determination of elements by ICP-AES and ICP-MS" by Henk J van de Wiel (National Institute of Public Health and the Environment (RIVM)) Bilthoven, The Netherlands (http://www.ecn.nl/docs/society/horizontal/hor desk 19 icp.pdf).

Levels of 7-epidocetaxel and 10-oxodocetaxel may be measured as follows; in accordance with US Pharmacopeia USP35, 2nd supplement, valid as from Dec 2012)

### Chromatographic system -

| | |
|---|---|
| Analytical column : | Waters Acquity UPLC BEH Phenyl, 1.7 µm, length 50 mm x |
| | |
| | 2.1 mm ID, or equivalent. |
| Mobile phase A : | Acetonitrile |
| | |
| Mobile phase B : | Dilute 0.5 mL of phosphoric acid (85%) to 2.0 L with Ultra Pure water (Milli-Q water is suitable) in a volumetric flask, mix and filter over a 0.2 µm membrane filter. |

| Gradient program : | Time (min.) | Mobile phase A (%) | Mobile phase B (%) |
|---|---|---|---|
| (Linear) | 0.0 | 19.5 | 80.5 |
| | 6.0 | 40.0 | 60.0 |
| | 9.0 | 40.0 | 60.0 |
| | 12.7 | 52.7 | 47.3 |
| | 13.1 | 94.3 | 5.7 |
| | 15.1 | 94.3 | 5.7 |
| | 15.8 | 19.5 | 80.5 |
| | 20.0 | 19.5 | 80.5 |

| | |
|---|---|
| Detection | : UV 227 nm |
| Injection volume | : 10 µL (full loop) |
| Flow | : 0.6 mL/min ± 0.1 mL/min |
| Sample temperature | : 5°C ± 3°C |
| Weak Needle Wash | : Solvent B |
| Strong Needle Wash | : Mixture of Acetonitrile/Ultra Pure water 80/20 (^{V}/_{V}) Seal |
| Wash | : Solvent B |
| Estimated Retention Time Docetaxel | : 6.5 min. |
| Estimated Relative Retention Time 10-DAC-Baccatin III | : 0.26 |
| Estimated Relative Retention Time Paclitaxel | : 1.09 |
| Estimated Relative Retention Time "Impurity RRT 1.09" | : 1.09 |
| Estimated Relative Retention Time 10-Oxodocetaxel | : 1.15 |
| Estimated Relative Retention Time 7-Epidocetaxel | : 1.17 |
| Estimated Relative Retention Time 7-Epi-10-oxodocetaxel | : 1.42 |

### Reference Example; Stability study of known Docetaxel solution shows degradation products.

One batch of each volume of 1-vial Taxotere® (20mg/ml and 80mg/ml) was subjected to an accelerated stability program (40 °C/75% RH) in order to establish the stability and the degradation profile. Both products are also tested at its respective expiry date (long term storage at 25 °C/60% RH). The results (Tables 1 and 2) show that the levels of 10-Oxo-docetaxel and 7-Epi-docetaxel can be expected to increase upon storage of the product. Furthermore, Impurity RRT 1.09 may be present after extended storage.

Both known degradation products are limited by the USP monograph for Docetaxel Injection at 1.5% and 0.5% respectively. Because of its unknown structure, Impurity RRT 1.09 should comply with the requirement for any unknown peak of nmt 0.2% (ref. USP35, 2nd supplement, valid as from Dec 2012).

**Table 1. 1-vial Taxotere® 20 mg/1 mL; accelerated study results (40°C/75%RH)**

| **Time point (months)** | **Initial** | **1** | **2** | **3** | **6** |
|---|---|---|---|---|---|
| pH | 3.9 | 3.8 | 3.8 | -- | 3.9 |

| Related substances (%) | | | | | |
|---|---|---|---|---|---|
| - 10-Deacetyl-baccatin III | n.d. | n.d. | <0.05 | n.d. | 0.06 |
| - Impurity RRT 1.09 | n.d. | 0.06 | 0.15 | 0.26 | 0.67 |
| - 10-Oxo-docetaxel | 0.19 | 0.37 | 0.57 | 0.75 | 0.96 |
| - 7-Epi-docetaxel | <0.05 | 0.08 | 0.15 | 0.23 | 0.44 |
| - 7-Epi-1 0-oxodocetacel | n.d. | n.d. | n.d. | 0.07 | 0.20 |
| - Largest unknown peak | 0.15 | 0.14 | 0.14 | 0.14 | 0.14 |
| - Total | 0.51 | 0.82 | 1.2 | 1.9 | 3.0 |
| Assay of Docetaxel (%) | 99.8 | 100.6 | 99.1 | 98.2 | 97.8 |
| Mass balance (%) | 100.3 | 101.4 | 100.3 | 100.1 | 100.8 |

**Table 2. 1-vial Taxotere® 80 mg/4 mL; accelerated study results(40°C/75%RH)**

| **Time point (months)** | **Initial** | **1** | **2** | **3** | **6** |
|---|---|---|---|---|---|
| pH | 3.8 | 3.8 | 3.8 | -- | 3.9 |

| Related substances (%) | | | | | |
|---|---|---|---|---|---|
| - 10-Deacetyl-baccatin III | <0.05 | n.d. | <0.05 | n.d. | <0.05 |
| - Impurity RRT 1.09 | n.d. | 0.06 | 0.13 | 0.22 | 0.64 |
| - 10-Oxo-docetaxel | 0.14 | 0.32 | 0.44 | 0.59 | 0.79 |
| - 7-Epi-docetaxel | <0.05 | 0.08 | 0.15 | 0.22 | 0.47 |
| - 7-Epi-1 0-oxodocetaxel | n.d. | n.d. | n.d. | <0.05 | 0.20 |
| - Largest unknown peak | 0.16 | 0.15 | 0.15 | 0.16 | 0.16 |
| - Total | 0.35 | 0.66 | 0.98 | 1.3 | 2.7 |
| Assay of Docetaxel (%) | 101.6 | 99.9 | 98.2 | 98.0 | 97.2 |
| Mass balance (%) | 102.0 | 100.6 | 99.2 | 99.3 | 99.9 |

| | | | | | |
|---|---|---|---|---|---|
| - - = not measured | | | | | |

### Example 1; Influence of residual alkali metals on stability

Polysorbate 80 usually contains residual amounts of the alkali metals Sodium and Potassium as a result of the manufacturing process. Purification can be conducted by removal of the alkali metals by cationic resin (Dowex monosphere 650C UPW) exchange treatment. This process decreases the pH of the Polysorbate 80 by introduction of H+ ions, which can be used as an indication of completion of the purification process. Experiments were conducted with resin treatment of Caldic PS80 Polysorbate 80 to various pHs. The remaining alkali metals amounts were analyzed by ICP-MS (inductively coupled plasma mass spectrometry). The results are presented in Table 3.

**Table 3. Results of resin treatment of Polysorbate 80**

| **Polysorbate 80 treatment** | **pH 7 (untreated)** | **treated to pH 5** | **treated to pH 4** | **treated to pH 3.5** |
|---|---|---|---|---|
| K+ (ppm) | 163 | 43 | 22 | 8 |
| Na+ (ppm) | 148 | 40 | 21 | 6 |

Experimental batches of 40 mg/mL solution of Docetaxel in Polysorbate 80 as the Injection Concentrate and anhydrous ethanol/water as the diluent were prepared with resin treated Caldic PS80 to different pHs in order to assess the influence of residual alkali metals on the stability of the formulation. The batches were subjected to an accelerated stability program at 40 °C/75% RH up to 3 months (regarded as representative for 24 months long term storage). The results are presented in Tables 4a and 4b. The study results show that alkali metal catalyzed generation of 7-Epi-docetaxel is adequately reduced by the use of resin treated Polysorbate 80. Cationic resin treatment endpoint should be indicated by a final pH requirement.

It was noted that at when the pH of the polysorbate reached a lower level, a second degradation product 10-oxo-docetaxel was generated (see Table 4b). This is caused by residual oxidizing substances, such as peroxides in the Polysorbate 80. As this seems to be a pH-dependent process, additional testing was conducted in order to reduce residual oxidizing substances in the Polysorbate 80. for more information.

**Table 4a. Residual alkali metal experiment; study results**

| **Polysorbate 80 quality** | **untreated** | | | **treated to pH 5** | | |
|---|---|---|---|---|---|---|
| K+ (ppm) | >100 | | | 41 | | |
| Na+ (ppm) | >100 | | | 37 | | |

| **Time points (mths)** | **0** | **1** | **3** | **0** | **1** | **3** |
|---|---|---|---|---|---|---|
| pH | 7.5 | 5.9 | - | 5.3 | 4.0 | 3.9 |

| Related substances (%) | | | | | | |
|---|---|---|---|---|---|---|
| - 10-Oxo-docetaxel | 0.2 | n.d. | - | 0.1 | 0.1 | 0.4 |
| *- 7-Epi-docetaxel* | *6.6* | *37.0* | - | *0.1* | *2.2* | *2.7* |
| - Total | 8.2 | 55.0 | - | 0.3 | 4.1 | 9.7 |
| Assay (%) | 89.9 | 26.9 | - | 99.3 | 92.5 | 85.9 |
| Mass balance (%) | 98.1 | 81.9 | - | 99.6 | 96.6 | 95.6 |

**Table 4b. Residual alkali metal experiment; study results**

| **Polysorbate 80 quality** | **treated to pH 4** | | | **treated to pH 3.5** | | |
|---|---|---|---|---|---|---|
| K+ (ppm) | 21 | | | <10 | | |
| Na+ (ppm) | 19 | | | <10 | | |

| **Time points (mths)** | **0** | **1** | **3** | **0** | **1** | **3** |
|---|---|---|---|---|---|---|
| pH | 4.2 | 3.8 | 3.6 | 3.8 | 3.7 | 3.5 |

| Related substances (%) | | | | | | |
|---|---|---|---|---|---|---|
| - 10-Oxo-docetaxel | <0.1 | 0.3 | 3.1 | 0.1 | 0.7 | 8.0 |
| *- 7-Epi-docetaxel* | *0.1* | *0.2* | *1.3* | <*0.1* | <*0.1* | *0.1* |
| - Total | 0.1 | 0.9 | 5.8 | 0.3 | 0.9 | 10.2 |
| Assay (%) | 98.2 | 96.2 | 92.3 | 98.5 | 96.2 | 88.2 |
| Mass balance (%) | 98.3 | 97.1 | 98.1 | 98.8 | 97.1 | 98.4 |

### Example 2; Influence of residual oxidizing substance on stability

Polysorbate 80 usually contains residual amounts of oxidizing substances as a result of the manufacturing process, expressed by the peroxide value. These oxidizing substances may cause degradation of Docetaxel into 10-Oxo-docetaxel at lower product pHs, as can be seen from previous experiments (refer to Table 4b). To reduce the residual oxidizing substances, polysorbate with low peroxide value should be used. To assess the influence of the oxidizing substances (e.g., peroxide value), experimental batches of the Example 1 40mg/ml compositions were prepared with different Polysorbate 80 qualities. Both qualities were pretreated with resin in order to reduce the alkali metals effects. The batches were subjected to a stability program at 25 °C/60% RH up to 6 months. The results are presented in Table 5. The study results show that polysorbate with low peroxide value adequately reduces the generation of 10-Oxo-docetaxel.

**Table 5. Residual oxidizing substances experiment; study results**

| **Polysorbate 80 quality** | **Normal** | | | **High purity** | | |
|---|---|---|---|---|---|---|
| Manufacturer | Caldic PS80 | | | Croda Tween™ 80HP LQ | | |
| Peroxide value | 3 | | | 0 | | |
| Resin purification | yes | | | yes | | |

| **Time points (mths)** | **0** | **3** | **6** | **0** | **3** | **6** |
|---|---|---|---|---|---|---|
| pH | 3.3 | 3.2 | 3.3 | 3.4 | 3.4 | 3.4 |

| Related substances (%) | | | | | | |
|---|---|---|---|---|---|---|
| - *10-Oxo-docetaxel* | *0.1* | *1.8* | *2.8* | *0.1* | *0.1* | *0.1* |
| - 7-Epi-docetaxel | <0.1 | <0.1 | <0.1 | <0.1 | <0.1 | <0.1 |
| - Total | 0.2 | 2.0 | 3.2 | 0.2 | 0.3 | 0.4 |
| Assay (%) | 99.3 | 98.5 | 96.8 | 98.3 | 98.6 | 97.6 |
| Mass balance (%) | 99.5 | 100.5 | 100.0 | 98.5 | 98.9 | 98.0 |

Further commercially available polysorbate 80 samples (all having a pH of around 6.0, indicating the presence of Na+ and K+ ions instead of H+ ions) were resin treated before batch preparation.

### Selected USP/NF Polysorbate 80 qualities

| **Supplier** | **Quality** | **Peroxide value** | **pH** |
|---|---|---|---|
| Croda Tween™ 80HP LQ | low peroxide value, pH 6 | 0.0 | 5.7 |
| Seppic Montanox PPI | low peroxide value, pH 6 | 0.0 | 6.0 |
| Seppic Montanox API | pH 6 | 2.3 | 6.3 |

Using the selected brands and qualities experimental batches were prepared. The batches were subjected to a long term (25 °C/60% RH) stability program (note that 6 months can be regarded as representative for 24 months long term storage). The results are presented in Table 6.

**Table 6. Polysorbate quality assessment; long term study results**

| **Polysorbate 80 quality** | **Resin treatment** | **Initial pH** | **7-Epi-docetaxel (%)** | | | **10-Oxo-docetaxel (%)** | | |
|---|---|---|---|---|---|---|---|---|
| | | | **0*** | **3*** | **6*** | **0*** | **3*** | **6*** |
| Croda Tween 80HP LQ #1 | yes | 3.9 | <0.1 | <0.1 | 0.1 | <0.1 | 0.1 | 0.1 |
| Croda Tween 80HP LQ #2 | yes | 3.9 | <0.1 | <0.1 | 0.1 | <0.1 | 0.1 | 0.2 |
| Seppic Montanox PPI | yes | 3.7 | <0.1 | 0.1 | 0.1 | <0.1 | 0.1 | 0.2 |
| Seppic Montanox API | yes | 4.0 | <0.1 | <0.1 | 0.1 | <0.1 | 0.2 | 0.4 |
| Seppic Montanox API | no | 6.3 | <0.1 | 3.6 | 6.6 | <0.1 | 0.5 | 0.4 |

| | | | | | | | | |
|---|---|---|---|---|---|---|---|---|
| * Time points in months | | | | | | | | |

### Example 3; Influence of Citric Acid

Taxotere® contains citric acid which has been demonstrated to have a positive influence on stability.

To assess the necessity of addition of Citric acid, experimental batches were prepared without Citric Acid and with addition of Citric Acid in 2 concentrations. The experimental batches were prepared with pre-processed (e.g., resin treated) Croda Tween^{™} 80HP LQ. The batches were subjected to an accelerated (40 °C/75% RH) stability program (note that 3 months can be regarded as representative for 24 months long term storage). See Table 7 for results, showing that addition of Citric Acid does not improve stability and therefore, is not crucial for the formulation and can therefore be excluded from the formulation.

**Table 7. Citric acid assessment; accelerated study results**

| **Citric acid addition** | **Initial pH** | **7-Epi-docetaxel (%)** | | | **10-Oxo-docetaxel (%)** | | |
|---|---|---|---|---|---|---|---|
| | | **0*** | **3*** | **6*** | **0*** | **3*** | **6*** |
| none | 3.9 | <0.1 | 0.1 | 0.2 | <0.1 | 0.1 | 0.2 |
| 0.5 mg/mL | 3.6 | <0.1 | 0.1 | 0.1 | <0.1 | 0.2 | 0.4 |
| 1 mg/mL | 3.4 | <0.1 | 0.1 | 0.1 | <0.1 | 0.2 | 0.4 |
| none | 3.9 | <0.1 | 0.1 | 0.1 | <0.1 | 0.1 | 0.3 |
| 0.5 mg/mL | 3.6 | <0.1 | 0.1 | 0.1 | <0.1 | 0.2 | 0.4 |
| 1 mg/mL | 3.4 | <0.1 | 0.1 | 0.1 | <0.1 | 0.2 | 0.4 |

| | | | | | | | |
|---|---|---|---|---|---|---|---|
| * Time points in months | | | | | | | |

### Example 4

Cabazitaxel compositions can be prepared as follows using resin-treated polysorbate 80 , optionally of low peroxide content;

Fill volume: 6 ml

50% ethanol abs / 50% Polysorbate 80

| **Component** | **Quality standard** | **Concentration (mg/ml)** | **Unit formula (mg/vial)** |
|---|---|---|---|
| Cabazitaxel | Company standard | 10 mg | 60 mg |
| Polysorbate 80 | Ph.Eur./USP/JP | 540 mg | 3240 mg |
| Ethanol abs. | Ph.Eur./USP/JP | 394.50 mg | 2367 mg |

## Claims

1. A pharmaceutical composition containing a taxoid and polysorbate 80 wherein the level of sodium ions and potassium ions present in the polysorbate 80 is each less than 25ppm, said composition optionally containing anhydrous alcohol, preferably anhydrous ethanol.

2. A pharmaceutical composition according to claim 1, wherein the level of sodium ions and potassium ions present in the polysorbate 80 is each less than 10ppm.

3. A pharmaceutical composition according to claim 1 or 2 wherein the pH of the polysorbate 80 is less than 5.

4. A pharmaceutical composition according to claim 3 wherein the pH of the polysorbate 80 is 3.8 or less, more preferably, 3.8.

5. A pharmaceutical composition according to any preceding claim, wherein the taxoid drug is docetaxel.

6. A pharmaceutical composition according to any of claims 1-4, wherein the taxoid drug is cabazitaxel.

7. A pharmaceutical composition according to any preceding claim, wherein the peroxide level of the polysorbate 80 between 2 and 0.1 or 0.

8. A pharmaceutical composition according to claim 5, wherein the level of 7-epi-docetaxel is 0.3% or less after storage for 6 months at 25°C and a relative humidity of 60%.

9. A pharmaceutical composition according to claim 8, wherein the level of 7-epi-docetaxel is 0.1% or less after storage for 6 months at 25°C and a relative humidity of 60%.

10. A pharmaceutical composition according to claim 7 when the taxoid drug is docetaxel, wherein after storage for 6 months at 25°C and a relative humidity of 60% the level of 7-epi-docetaxel is 0.3% or less and the level of 10-oxo-docetaxel is 0.4% or less.

11. A pharmaceutical composition according to claim 10, wherein the level of 7-epi-docetaxel is 0.1 or less and the level of 10-oxo-docetaxel is 0.2 or less after storage for 6 months at 20°C and a relative humidity of 60%.

12. A pharmaceutical composition according to any preceding claim, further containing citric acid.

13. A pharmaceutical composition according to any preceding claim, wherein the pH of the composition is from 3.0 to 4.5.

14. A stable pharmaceutical composition containing docetaxel that is free of citric acid and other pH reducing agents, said composition optionally containing anhydrous alcohol, preferably anhydrous ethanol.

15. A stable pharmaceutical composition containing cabazitaxel that is free of citric acid and other pH reducing agents, said composition optionally containing anhydrous alcohol, preferably anhydrous ethanol.

16. Pharmaceutical composition containing docetaxel and polysorbate, wherein said polysorbate 80 has a pH of less than 5.

17. A composition according to any of claims 14-16, wherein said polysorbate 80 has a pH 3.8.

18. A composition according to any of claims 14-17, wherein the level of sodium ions and potassium ions present in the polysorbate 80 is each less than 25ppm, more preferably each less than 10ppm.

19. A composition according to any of claims 14-18, wherein the polysorbate 80 has a peroxide level between 0 or 0.1 and 2.0.

20. A composition according to any of claims 14-18, wherein the level of 7-epi-docetaxel is 0.3% or less after storage for 6 months at 20°C and a relative humidity of 60%.

21. A composition according to claim 20 wherein the level of 7-epi-docetaxel is 0.1 % or less after storage for 6 months at 20°C and a relative humidity of 60%.

22. A composition according to claim 19, wherein upon storage the level of 7-epi-docetaxel is 0.3% or less and the level of 10-oxo-docetaxel is 0.4% or less after storage for 6 months at 20°C and a relative humidity of 60%.

23. A composition according to claim 22, wherein the level of 7-epi-docetaxel is 0.1% or less and the level of 10-oxo-docetaxel is 0.2% or less after storage for 6 months at 20°C and a relative humidity of 60%.

24. A process of preparing a pharmaceutical composition as defined in any preceding claim comprising;
a. exposing polysorbate 80 to cation exchange resin,
b. filtering said mixture to obtain polysorbate 80 with a reduced level of sodium and potassium ions
c. combining said polysorbate 80 with docetaxel or cabazitaxel optionally dissolved in ethanol.

25. A process according to claim 24, wherein the polysorbate 80 is exposed to resin until the pH of the polysorbate is pH 5.0 or less.

26. A process according to claim 25, wherein the polysorbate 80 is exposed to resin until the pH of the polysorbate is pH 3.8.

27. A process according to any of claims 24-26, wherein the peroxide level of the polysorbate 80 between 2 and 0.1 or 0.

28. A process according to any of claims 24-27, for preparing a stable pharmaceutical composition containing docetaxel (or cabazitaxel) that is free of citric acid (or similar pH reducing agents).

29. A process according to any of claims 24-28, wherein the pH of the composition is from 3.0 to 4.5.

30. A pharmaceutical composition comprising docetaxel (or cabazitaxel) and polysorbate 80 wherein said polysorbate 80 is substantially free of sodium and potassium ions.

31. A pharmaceutical composition according to claim 30, wherein said composition contains less than 10ppm sodium ions and less than 10ppm potassium ions.
